# EUROPEAN PATENT APPLICATION

(11) **EP 2 506 171 A1**
(43) Date of publication of application: **03.10.2012**
(21) Application number: 11002763.8
(22) Date of filing: 01.04.2011
(51) Int. Cl.: G06F 19/00, G06Q 10/00

(54) **Graphical user interfaces for scientific data information sytems**

(71) Applicant: Waters Technologies Corporation, Milford, MA 01757 (US)
(72) Inventor: Threlfall, Richard Steven, WA 15 8LF Hale, Altrincham Cheshire (GB); Crosidale, Scott, WF14 9QY Mirfiled (GB); Wintergalen, Andreas, Dr., 50529 Pulheim (DE)
(74) Representative: Vossius, Corinna

(57) **Abstract**

A method for assisting a user with interacting with laboratory data in a scientific data information system. The method includes displaying a graphical user interface having a sub-item navigation bar including a plurality of selectable tabs each associated with a corresponding one of a plurality of categories, and simultaneously displaying in the graphical user interface a main work area containing information about a selected one of the categories. The user can toggle from one category to another with a single input action.

## Description

### TECHNICAL FIELD

This disclosure relates to scientific data information systems, and in particular to graphical user interfaces for scientific data information systems.

### BACKGROUND

Modem science-driven organizations, such as biotechnology and pharmaceutical companies, face intense scientific, regulatory, and business challenges. As both regulatory (e.g., GxP) scrutiny and scientific complexity increase, companies are often forced to demonstrate in even greater detail that their product (e.g., biologic/pharmaceutical product) is well-characterized and that their production process is well-controlled. For example, GxP compliance requires a system of administrative and information access controls, as well as audit trails of user activities and record alterations.

At the heart of the challenge of operating in a regulated (e.g., GxP) environment is a pressure to capture as much orthogonal information as possible during the development process and leverage it in manufacturing and quality control.

With the continual pressure to capture high-quality data, and do more with it, each functional area within a science-driven organization, and the organization as a whole, could benefit from being able to access and process captured data more efficiently.

### SUMMARY

In one aspect, the invention provides a method for assisting a user with interacting with laboratory data in a scientific data information system. The method includes displaying a graphical user interface having a sub-item navigation bar including a plurality of selectable tabs each associated with a corresponding one of a plurality of categories, and simultaneously displaying in the graphical user interface a main work area containing information about a selected one of the categories. The user can toggle from one category to another with a single input action.

In another aspect, the invention provides a method for assisting a user with interacting with laboratory data in a scientific data information system. The method includes displaying a graphical user interface having a sub-item navigation bar including a plurality of selectable tabs each associated with a corresponding one of a plurality of categories, simultaneously displaying in the graphical user interface a main work area containing information about a selected one of the categories, and simultaneously displaying in the graphical user interface a task panel containing links to tasks associated with laboratory work and/or links to recently viewed items.

A further aspect of the invention features a computer-readable medium having stored thereon computer-executable instructions for displaying, on a display device, a graphical user interface having a sub-item navigation bar including a plurality of selectable tabs each associated with a corresponding one of a plurality of categories, and simultaneously displaying in the graphical user interface a main work area containing information about a selected one of the categories. The user can toggle from one category to another with a single input action.

Another aspect of the invention features a computer-readable medium having stored thereon computer-executable instructions for displaying a graphical user interface having a sub-item navigation bar including a plurality of selectable tabs each associated with a corresponding one of a plurality of categories, simultaneously displaying in the graphical user interface a main work area containing information about a selected one of the categories, and simultaneously displaying in the graphical user interface a task panel containing links to tasks associated with laboratory work and/or links to recently viewed items.

Implementations may include one or more of the following features.

In some implementations, the plurality of selectable tabs includes a current work tab associated with a current work category, a recent work tab associated with a recent work category, an instrument systems tab associated with an instrument systems category, and a reports to sign tab associated with a reports to sign category.

In certain implementations, a list of current work items representing laboratory work currently being run by the user and/or by members of an associated work group is displayed in the main work area in response to a signal indicating user selection of the current work tab. In some cases, the list of current work items corresponds to content stored within a database.

In some implementations, a database is maintained. Maintaining the database can include storing information corresponding to the current work items within a service-side work area within the database, and, upon completion of one of the work items, storing information corresponding to the completed work item in a unified data structure within the database. The service-side work area is distinct from the unified data structure.

In certain implementations, a list of one or more completed work items, each corresponding to an associated unified data structure stored within the database, is displayed in the main work area in response to a signal indicating user selection of the recent work tab.

In some implementations, the database is maintained in a manner compliant with GxP requirements.

In certain implementations, a list of one or more completed work items representing laboratory work that has been completed by the user and/or by members of an associated work group is displayed in the main work area in response to a signal indicating user selection of the recent work tab. Information shown in the list of completed work items can include analysis results and methods (e.g., analysis methods).

In some implementations, an option, e.g., a drop-down list, allowing the user to select a time span for the list of completed work items being displayed is presented in the main work area.

In certain implementations, a list of laboratory instruments available to the user and/or to members of an associated work group is displayed in the main work area in response to a signal indicating user selection of the instrument systems tab.

In some implementations, a list of work items that are awaiting electronic signature by the user or a member of an associated workgroup is displayed in the main work area in response to a signal indicating user selection of the reports to sign tab.

In some implementations, a master list area with a list of work items that are awaiting electronic signature by the user or a member of an associated workgroup, and a detail area displaying a signature status overview and signature history for a selected one of the work items from the master list area, is displayed in the main work area in response to a signal indicating user selection of the reports to sign tab.

In some implementations, the plurality of selectable tabs comprise a current work tab associated with a current work category, a recent work tab associated with a recent work category, an instrument systems tab associated with an instrument systems category, and a reports to sign tab associated with a reports to sign category.

In some implementations, the computer-readable medium further comprising computer-executable instructions for displaying in the main work area a list of current work items representing laboratory work currently being run by the user and/or by members of an associated work group when the current work tab is selected.

In some implementations, the computer-readable medium further comprising computer-executable instructions for displaying in the main work area a list of one or more completed work items representing laboratory work that has been completed by the user and/or by members of an associated work group when the recent work tab is selected.

In some implementations, information shown in the list of completed work items includes analysis results and methods.

In some implementations, the computer-readable medium further comprising computer-executable instructions for presenting in the main work area an option allowing the user to select a time span for the list of completed work items being displayed.

In some implementations, the option is presented as a drop-down list.

In some implementations, the computer-readable medium further comprising computer-executable instructions for displaying in the main work area a list of laboratory instruments available to the user and/or to members of an associated work group when the instrument systems tab is selected.

In some implementations, the computer-readable medium further comprising computer-executable instructions for displaying in the main work area a list of work items that are awaiting electronic signature by the user or a member of an associated workgroup when the reports to sign tab is selected.

In some implementations, the computer-readable medium further comprising computer-executable instructions for displaying in the main work area a master list area with a list of work items that are awaiting electronic signature by the user or a member of an associated workgroup, and a detail area displaying a signature status overview and signature history for a selected one of the work items from the master list area.

Other aspects, features, and advantages are in the description, drawings, and claims.

### BRIEF DESCRIPTION OF THE DRAWINGS

FIG. 1 is a schematic view of a scientific data information system.
FIG. 2 is a schematic view of a workstation model including the scientific data information system of FIG. 1.
FIG. 3 is a computer network including the scientific data information system of FIG. 1.
FIG. 4 is a screen shot illustrating a graphical user interface of the scientific data information system of FIG. 1
FIG. 5 is a screen shot illustrating user selection of a "Current Work" category of the graphical user interface of FIG. 4.
FIG. 6 is a screen shot illustrating user selection of a "Recent Work" category of the graphical user interface of FIG. 4.
FIG. 7 is a screen shot illustrating user selection of a "Reports to Sign" category of the graphical user interface of FIG. 4.

Like reference numbers indicate like elements.

### DETAILED DESCRIPTION

A scientific data information system can be provided with a graphical user interface that provides a user with an option to access almost all relevant information and links necessary to start the daily laboratory work with zero or 1 input action (e.g., mouse click or keystroke). Such a user interface can help to increase user efficiency and productivity as relevant information and tasks are readily accessible.

### I. System Overview

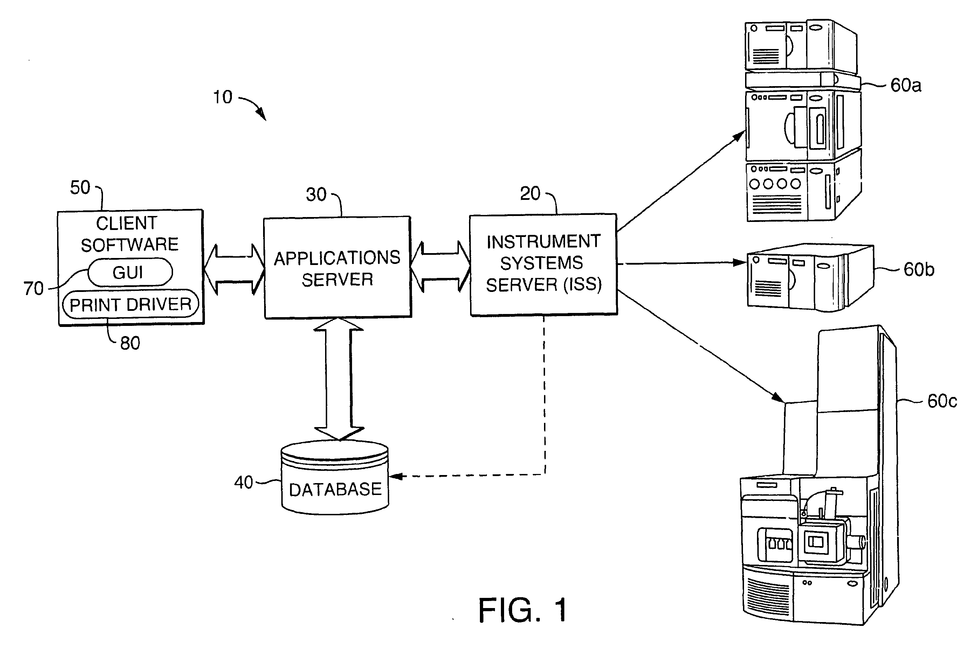

FIG. 1 illustrates a scientific data information system 10 that provides for acquisition of chromatography and mass spectrometry data (within a single software environment), instrument control, data processing and mining, and reporting, with GxP laboratory compatibility that allows for deployment throughout a science-driven organization. The scientific data information system includes an instrument systems server (ISS) 20, an application server 30, a database 40, and client software 50.

The ISS 20 is in communication with laboratory instruments 60a-c and includes computer-executable instructions for handling instrument control and data acquisition. The laboratory instruments 60a-c can include, for example, chromatographic instruments 60a, detectors 60b (e.g., UV detectors), and mass spectrometers 60c. Exemplary chromatographic instruments include ACQUITY UPLC® H-Class Bio System, available from Waters Corporation of Milford, Massachusetts. Exemplary detectors include the ACQUITY UPLC® Tunable UV (TUV) Detector, available from Waters Corporation. Exemplary mass spectrometers include the Xevo® G2 Tof mass spectrometer, available from Waters Corporation.

Generally, the ISS 20 performs two functions (i) system coordination, and (ii) data buffering. The ISS 20 can coordinate operation of the instruments based on information (e.g., instrument method and sample set information) received from the application server 30, which allows the ISS 20 to set up the instruments and start an acquisition. Instrument methods include instructions for controlling operating parameters of one of an attached instrument. The ISS 20 also provides status information back to the application server 30 during a run.

During data acquisition, the acquired data (e.g., chromatographic and/or mass spectrometry (MS) data) is received by the ISS 20 from the laboratory instruments 60a-c in native instrument format. The data is then translated by the ISS 20 to unified datafile format. Converted data is stored by the ISS 20 in a secure file buffer, and a rolling SHA1 checksum, incremented with each data packet, ensures fidelity and security of data. A final checksum is calculated upon acquisition completion and the raw data file is delivered to the database 40 where it is stored and locked.

The application server 30 is in communication with the ISS 20, the database 40, and the client software 50. The application server 30 handles the business logic (i.e., the functions that the associated software performs on the data). The application server 30 retrieves data (from the database 40), processes and presents data to a graphical user interface 70, processes input data (e.g., from the graphical user interface 70), and sends method (e.g., instrument method) and sample set information to the ISS 20 to set up the instruments and start an acquisition. In addition, the application server 30 and the ISS 20 communicate on a host of configuration and setup issues, such as downloading instrument drivers to the ISS 20, configuring instrument systems, etc. This is driven from the application server 30 to the ISS 20.

The application server 30 includes computer-executable instructions for providing administrative and information access controls, as well as for providing audit trails of user activities and record alterations in accordance with GxP compliance requirements. Each unique user has tunable information access (method, data, results, etc.) limitations and activity restrictions dictated by their assigned roles. Users can include administrators, managers, analysts, and principal scientists.

The application server 30 also includes computer-executable instructions for performing data processing, e.g., to reduce the raw data acquired from the laboratory instruments 60a-c into usable reports. Data (e.g., chromatographic data, spectral (MS) data, and bioinformatics) can be processed, by the application server 30, while acquisition is ongoing if processing parameters are specified within a method (e.g., an analysis method). Analysis methods can describe expected system hardware configurations, separation and MS parameters, spectral processing and bioinformatics analysis tasks, and links to automated reporting templates, which can be used to automate production of standardized reports. Following data collection, a copy of the corresponding analysis method can be stored as part of each results set. The application server 30 also relays information, e.g., method and sample set information, to the ISS 20, which then controls the instruments 60a-c according to the information provided.

The database 40 is a relational database. Relational databases enable real-time acquisition, processing, and management of large volumes of data from multiple sources. This can allow for simultaneous processing, review, and acquisition of data and parallel data acquisition from multiple instrument systems. Suitable relational databases include the Oracle® 11gR2 relational database, available from Oracle Corporation of Redwood Shores, California. Information stored in the database 40 can include many different data types (e.g., analyses, raw data, reports, historical data, methods, etc.) which may be stored in a unified data structure (also referred to as a "Content Item"). The use of a unified data structure can help to enable all laboratory functions to work with a common backbone of analytical information. This data standardization can also help to increase the exchange of information within an organization (e.g., between product development and product manufacturing), and, in some cases, even globally (e.g., with third-party partners).

The client software 50 includes computer-executable instructions (e.g., a Windows Presentation Foundation (or WPF) piece of code) for providing the graphical user interface 70 which displays data and allows the user to interact with the data (via the application server 30). Users can use the graphical user interface to select/define methods (e.g., instrument methods, analysis methods, capture methods, signature methods), to process data, and electronically review and sign reports. When the user decides to process data, instructions are sent the application server 30, where the processing takes place.

The client software 50 also includes a print driver 80 for performing print capture. The software generates a print file and moves the file through the application server 30 and stores it on the database 40. The print capture feature can be used for brining in auxiliary information into the system.

These software components (also known as computer programs, programs, software, software applications or code) include machine instructions for a programmable processor. As used herein, the terms machine-readable medium and computer-readable medium refer to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term machine-readable signal refers to any signal used to provide machine instructions and/or data to a programmable processor.

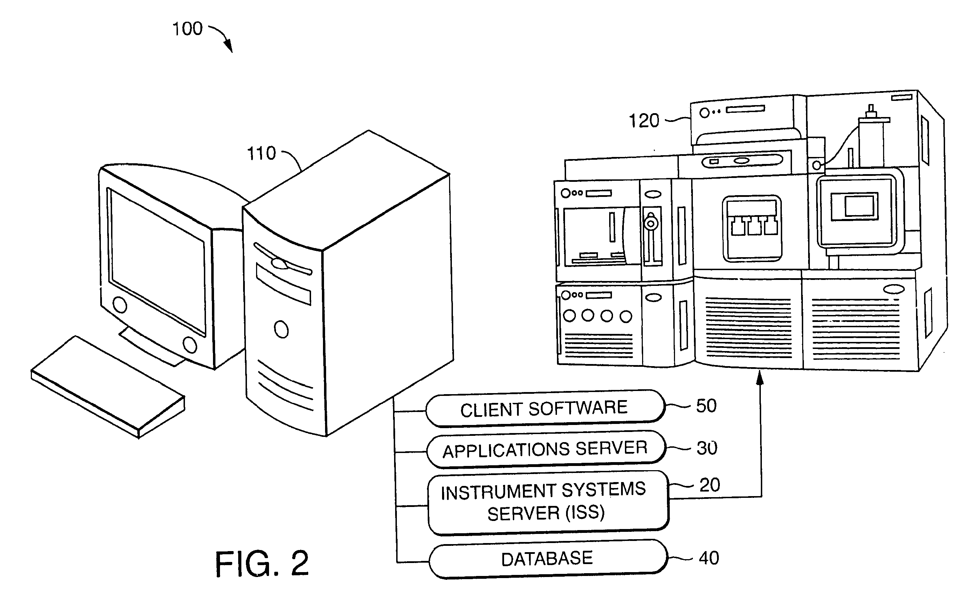

The scientific data information system 10 can be implemented in a variety of configurations, from an individual workstation model, to a network model, such as a laboratory-based workgroup or networked enterprise environment. In a workstation model, one computer handles both the low-level (e.g., database management and instrument control) and high level (e.g., data processing and user interface) functions. For example, FIG. 2 illustrates a workstation 100 in which the client software 50, the application server 30, the ISS 20, and the database 40 all reside on a single computer 110. A suitable computer 110 for the workstation 100 is a Lenovo D20 Workstation configured with dual Xeon E5504 2.0 GHz processors, 8 GB RAM, Nvidia Quadro FX 18000 graphics card under the Windows 7 64-bit operating system. The workstation 100 can also include a key board and a pointing device (e.g., a mouse or a trackball) for receiving user input, and a display device for displaying the graphical user interface 70. The workstation 100 can be physically located next to a laboratory instrument 120, such as an liquid chromatography (LC)/mass spectrometry (MS) system.

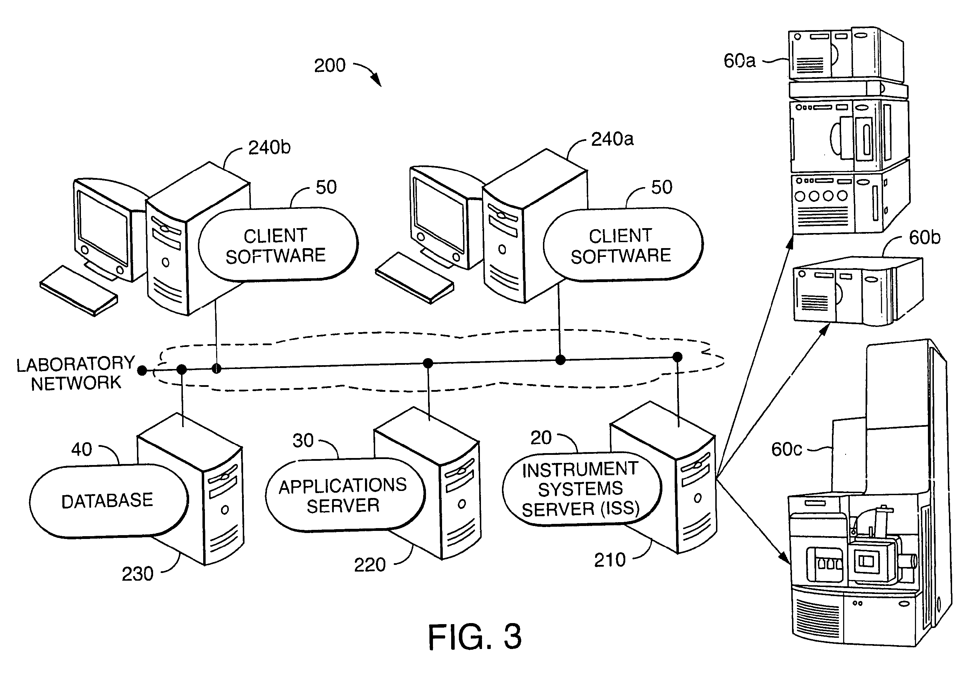

In a network model, the user interface, data processing, database management and instrument control functions can be split across separate computers which may be connected over a computer network, e.g., such as a local area network (LAN), wide area network (WAN), the Internet, or a combination thereof. For example, FIG. 3 illustrates a network 200 that includes an information system computer 210, an application server computer 220, a database management computer 230 and one or more client PC's 240a, 240b on which the ISS 20, the application server 30, the database 40, and the client software 50 reside, respectively. In some cases, such as in a laboratory-based workgroup, the application server and the database may reside on a common computer.

Each of the network computers can include a processor for processing instructions (e.g., stored in memory or on a storage device) for execution within the corresponding computer; a memory (e.g., volatile memory, non-volatile memory, a magnetic disk, an optical disk, etc.) for storing information within the corresponding computer; and a storage device (e.g., a floppy disk device, a hard disk device, an optical disk device, or a tape device, a flash memory, etc.) for providing mass storage for the corresponding computer.

### II. The Graphical User Interface

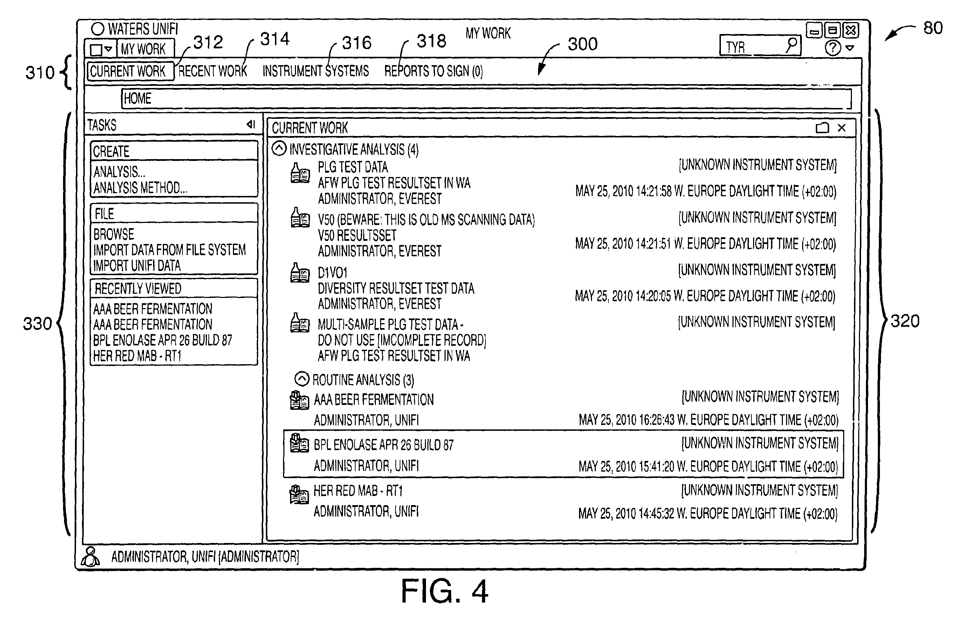

For a user starting daily work in the laboratory it can be helpful to have an immediate overview of all ongoing work and new tasks. It can also be beneficial to have an instant indication of any unexpected events (e.g., failure of laboratory instruments) that might have an impact on the user's work. In this regard, with reference to FIG. 4, the graphical user interface 70 includes a My Work page 300, which provides the user with an option to access all relevant information and links to start daily work in a laboratory with zero or one input action (e.g., a mouse click or a keystroke).

Referring to FIG. 4, the My Work page 300 includes a sub-item navigation bar 310, a Main Work area 320, and a Task panel 330. The sub-item navigation bar 310 allows the user to select categories, represented by tabs 312, 314, 316, 318 in FIG. 4. The tabs/categories include Current Work 312, Recent Work 314, Instrument Systems 316, and Reports to Sign 318. The user can toggle from one category to another with a single input action (e.g., a single mouse click or a single keystroke).

The Main Work area 320 contains information about the selected category. The Task panel 330 contains links to tasks relevant to the daily laboratory work and links to recently viewed items (e.g., analyses, analysis methods, reports, instrument systems, etc.).

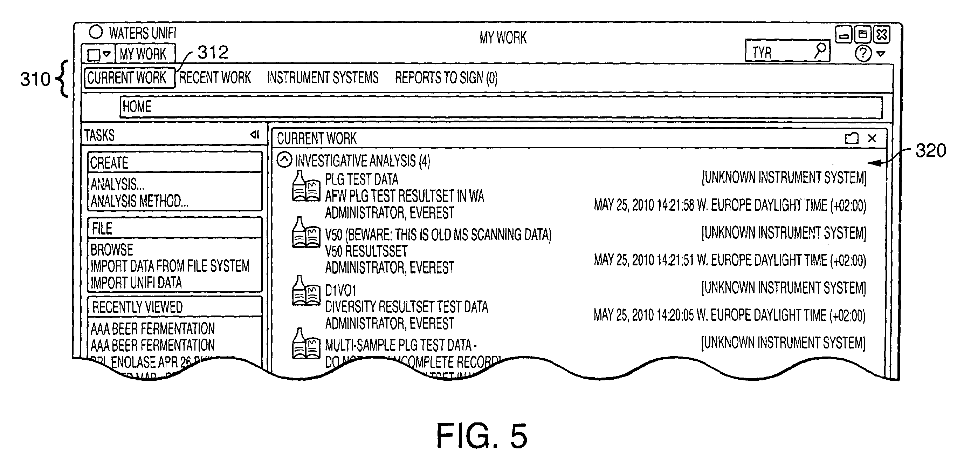

Referring to FIG. 5, when the Current Work tab 312 is selected, the Main Work area 320 displays the work that the user or members of the user's workgroup are currently running. Typical work items shown in this list can include ongoing analyses and analysis methods that the user is currently working on. The work shown is determined by the contents of a service-side Work Area.

The service-side Work Area is a user-protected, private back-up of working data - the analysis that the user is currently working on, and it is maintained within the database 40 (FIG. 1). When the user creates an analysis, it is created in the service-side Work Area. As it can take considerable time for an analysis to complete (e.g., hours/days/week), it is kept as "private" data for the user until it is completed. Once the user is satisfied with the analysis, the user can "complete" the analysis and publish it to the world. At this point, it is moved to a Content Item within the database 40.

Snapshots of an ongoing analysis are frequently taken and stored in the service-side Work Area as a back-up. This allows for extended workflows that can span powering down the machine (or software crashes, should that ever occur). In this regard, the service-side Work Area can help to avoid the loss of intermediate work in cases of network or server crashes (or at least minimize the lost work to a few minutes), and can provide the ability to restore a previously saved work record. The service-side Work Area can also allow a user to stop work in an unfinished form and continue at a later time, or to continue work in a different location and/or on a different computer (e.g., a different thin Client PC on a common network), without making the intermediate work product public.

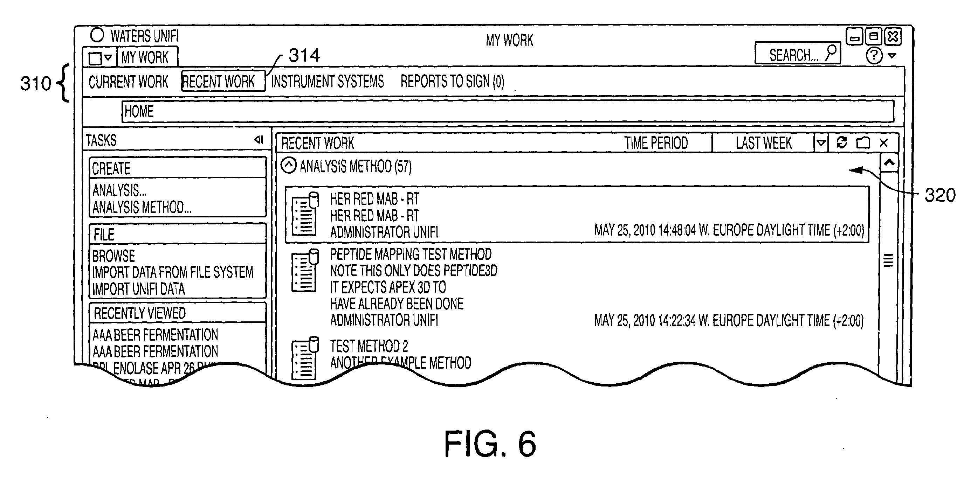

An added benefit is that the service-side Work Area allows the user to clearly see (via the Current Wok sub item on the user interface 70) what items the user is currently working on, as the user may have several analysis open simultaneously. Referring to FIG. 6, when the Recent Work tab 314 is selected, the Main Work area 320 displays work that the user or members of the user's workgroup have completed. Completed work entries are items that have been moved from the service-side Work Area and saved to content, as content items, within the database. This may also include other items saved to content that are deemed important to display as analysis results and analysis methods. It is possible to determine the time span (e.g., day, week, month, etc.) for work being displayed from a predefined selection. For example, a drop-down list 322 may be presented in the Main Work area 320 allowing the user to select a time span for the work being displayed.

When the Instrument Systems tab 316 is selected, the Main Work area 320 displays a stylized list of instruments available to either the user or to the user's workgroup.

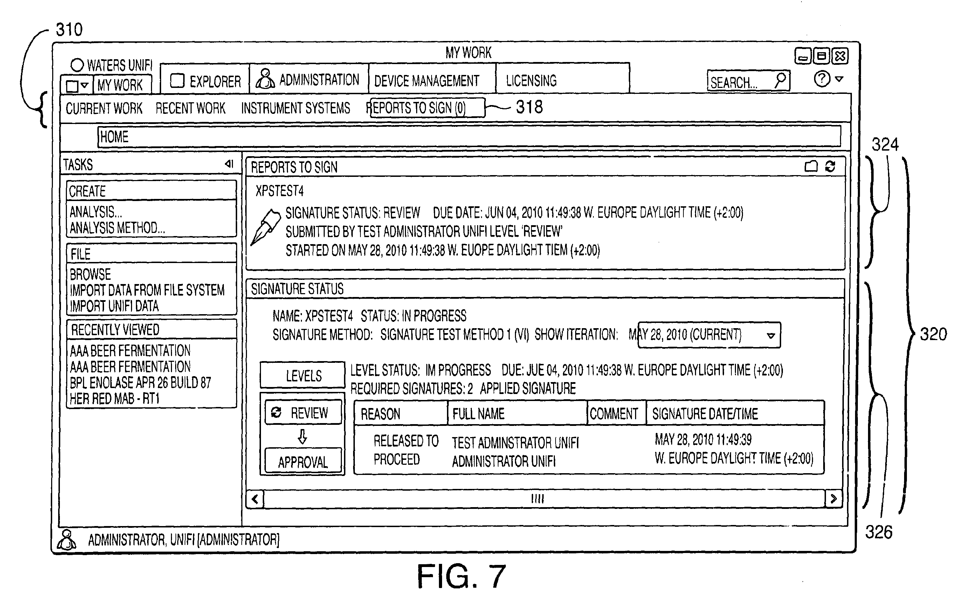

Referring to FIG. 7, when the Reports to Sign tab 318 is selected, the Main Work area 320 displays a stylized list of work items that are awaiting an electronic signature by either the user or the user's workgroup. The Main Work area 320 can, for example, include a master-detail type layout with the list of work items presented in a master list area 324 and with additional details of a selected one of the listed work items presented in a bottom "detail" area 326. The detail area 326 can, for example, display a signature status overview/signature history for the selected one of the work items from the master list area 324.

Although a few implementations have been described in detail above, other implementations are within the scope of the following claims.

## Claims

1. In a scientific data information system, a method for assisting a user with interacting with laboratory data, the method comprising:
displaying a graphical user interface having a sub-item navigation bar including a plurality of selectable tabs each associated with a corresponding one of a plurality of categories, and
simultaneously displaying in the graphical user interface a main work area containing information about a selected one of the categories,
wherein the user can toggle from one category to another with a single input action.

2. The method of claim 1, wherein the plurality of selectable tabs comprise a current work tab associated with a current work category, a recent work tab associated with a recent work category, an instrument systems tab associated with an instrument systems category, and a reports to sign tab associated with a reports to sign category.

3. The method of claim 1 or claim 2, in particular of claim 2, further comprising:
displaying in the main work area a list of current work items representing laboratory work currently being run by the user and/or by members of an associated work group in response to a signal indicating user selection of the current work tab.

4. The method of any one of claims 1 to 3, in particular of claim 3, wherein the list of current work items corresponds to content stored within a database.

5. The method of any one of claims 1 to 4, in particular of claim 4, further comprising:
maintaining a database including:
storing information corresponding to the current work items within a service-side work area within the database, and
upon completion of one of the work items, storing information corresponding to the completed work item in a unified data structure within the database, wherein the service-side work area is distinct from the unified data structure.

6. The method of any one of claims 1 to 5, in particular of claim 5, further comprising:
displaying in the main work area a list of one or more completed work items each corresponding to an associated unified data structure stored within the database in response to a signal indicating user selection of the recent work tab.

7. The method of any one of claims 1 to 6, in particular of claim 2, further comprising:
displaying in the main work area (i) a master list area with a list of work items that are awaiting electronic signature by the user or a member of an associated workgroup, and (ii) a detail area displaying a signature status overview and signature history for a selected one of the work items from the master list area, in response to a signal indicating user selection of the reports to sign tab.

8. In a scientific data information system, a method for assisting a user with interacting with laboratory data, the method comprising:
displaying a graphical user interface having a sub-item navigation bar including a plurality of selectable tabs each associated with a corresponding one of a plurality of categories,
simultaneously displaying in the graphical user interface a main work area containing information about a selected one of the categories, and
simultaneously displaying in the graphical user interface a task panel containing links to tasks associated with laboratory work and/or links to recently viewed items.

9. A computer-readable medium having stored thereon computer-executable instructions for:
displaying, on a display device, a graphical user interface having a sub-item navigation bar including a plurality of selectable tabs each associated with a corresponding one of a plurality of categories, and
simultaneously displaying in the graphical user interface a main work area containing information about a selected one of the categories,
wherein the user can toggle from one category to another with a single input action.

10. The computer-readable medium of claim 9, wherein the plurality of selectable tabs comprise a current work tab associated with a current work category, a recent work tab associated with a recent work category, an instrument systems tab associated with an instrument systems category, and a reports to sign tab associated with a reports to sign category.

11. The computer-readable medium of claim 9 or claim 10, in particular of claim 10, further comprising computer-executable instructions for displaying in the main work area a list of current work items representing laboratory work currently being run by the user and/or by members of an associated work group when the current work tab is selected.

12. The computer-readable medium of any one of claims 9 to 11, in particular of claim 10, further comprising computer-executable instructions for displaying in the main work area a list of one or more completed work items representing laboratory work that has been completed by the user and/or by members of an associated work group when the recent work tab is selected.

13. The computer-readable medium of any one of claims 9 to 12, in particular of claim 12, further comprising computer-executable instructions for presenting in the main work area an option allowing the user to select a time span for the list of completed work items being displayed.

14. The computer-readable medium of any one of claims 9 to 13, in particular of claim 10, further comprising computer-executable instructions for displaying in the main work area a list of laboratory instruments available to the user and/or to members of an associated work group when the instrument systems tab is selected.

15. The computer-readable medium of any one of claims 9 to 14, in particular of claim 10, further comprising computer-executable instructions for displaying in the main work area a list of work items that are awaiting electronic signature by the user or a member of an associated workgroup when the reports to sign tab is selected.

16. The computer-readable medium of any one of claims 9 to 15, in particular of claim 10, further comprising computer-executable instructions for displaying in the main work area a master list area with a list of work items that are awaiting electronic signature by the user or a member of an associated workgroup, and a detail area displaying a signature status overview and signature history for a selected one of the work items from the master list area.

17. A computer-readable medium having stored thereon computer-executable instructions for:
displaying a graphical user interface having a sub-item navigation bar including a plurality of selectable tabs each associated with a corresponding one of a plurality of categories,
simultaneously displaying in the graphical user interface a main work area containing information about a selected one of the categories, and
simultaneously displaying in the graphical user interface a task panel containing links to tasks associated with laboratory work and/or links to recently viewed items.
